(19) **Europäisches Patentamt**

**European Patent Office**

**Office européen des brevets**

(11) **EP 0 669 129 B1**

(12) **EUROPÄISCHE PATENTSCHRIFT**

(45) Veröffentlichungstag und Bekanntmachung des Hinweises auf die Patenterteilung:
**31.10.2001 Patentblatt 2001/44**

(51) Int Cl.⁷: **A61K 9/20**, A61K 9/50, A61K 9/16, A61K 9/00

(21) Anmeldenummer: **95102074.2**

(22) Anmeldetag: **15.02.1995**

(54) **Expandierbare Arzneiformen mit kontrollierter Wirkstoffabgabe**

Expandable release controlled medicaments

Médicaments expansibles à libération contrôlée

(84) Benannte Vertragsstaaten:
**AT BE CH DE DK ES FR GB GR IE IT LI LU MC NL PT SE**
Benannte Erstreckungsstaaten:
**LT SI**

(30) Priorität: **28.02.1994 DE 4406424**

(43) Veröffentlichungstag der Anmeldung:
**30.08.1995 Patentblatt 1995/35**

(73) Patentinhaber: **BAYER AG**
**51368 Leverkusen (DE)**

(72) Erfinder:
• **Penners, Gunther, Dr.**
  **D-51373 Leverkusen (DE)**
• **Lustig, Klemens, Dr.**
  **D-42115 Wuppertal (DE)**
• **Petersen-von-Gehr, Jörg, Dr.**
  **D-44795 Bochum (DE)**

(56) Entgegenhaltungen:
EP-A- 0 250 038          WO-A-93/00889
US-A- 5 158 777          US-A- 5 167 964

• DATABASE WPI Week 9422 Derwent Publications Ltd., London, GB; AN 94-178692 & JP-A-06 024 959 (BAYER YAKUHIN K.K.) , 1.Februar 1994
• DATABASE WPI Week 7732 Derwent Publications Ltd., London, GB; AN 77-56485Y & JP-A-52 076 418 (MICROBIAL CHEM. RES.) , 27.Juni 1977
• DATABASE WPI Week 8714 Derwent Publications Ltd., London, GB; AN 87-099083 & JP-A-62 048 618 (ZERIA SHINYAKU KOGY) , 3.März 1987
• DATABASE WPI Week 8804 Derwent Publications Ltd., London, GB; AN 88-024371 & JP-A-62 283 919 (ROLLER JAPAN K.K.) , 9.Dezember 1987

Bemerkungen:
Die Akte enthält technische Angaben, die nach dem Eingang der Anmeldung eingereicht wurden und die nicht in dieser Patentschrift enthalten sind.

EP 0 669 129 B1

**Beschreibung**

Die vorliegende Erfindung betrifft Mischungen aus Polyvinyllactamen und Polyacrylaten bei der Bereitstellung pharmazeutischer Systeme mit kontrollierter Wirkstoff-Freisetzung, die sich dadurch kennzeichnen, daß sie in der wäßrigen Umgebung des Magens stark quellen und über längere Zeit im Magen verweilen. Gegebenenfalls kann die Magenverweilzeit des erfindungsgemäßen Systems auch durch zusätzliches Einarbeiten einer gasbildenden Mischung beeinflußt werden, da das entstehende Gas die Dichte des Systems herabsetzt, wodurch es auf den Mageninhalt schwimmt und somit schlecht zum tiefer gelegenen Pförtner gelangen kann.

Die Verabreichung einer Einzeldosis eines Medikamentes, aus der der Wirkstoff über einen verlängerten Zeitraum kontrolliert freigesetzt wird (Retard Formulierung), hat im Vergleich zur Verabreichung einer Anzahl von schnell freisetzenden Einzeldosen zu bestimmten Intervallen den Vorteil, daß über einen verlängerten Zeitraum ein beständiger und einheitlicher Blutspiegel des Wirkstoffes gewährleistet wird. In der Pharmazie sind Retard Formulierungen der unterschiedlichsten Art bekannt. So gibt es beispielsweise Retard Formulierungen, die auf der kontrollierten Erosion einer wirkstoffhaltigen Matrix beruhen oder solche, aus denen ein wasserlöslicher Wirkstoff durch kontrollierte Diffusion durch eine oder mehrere die Formulierung umgebende Polymerschichten freigesetzt wird. In einer anderen retardiert freisetzenden Darreichungsform beruht die kontrollierte Freisetzung auf eine durch osmotische Wasseraufnahme bedingte Verdrängung einer wirkstoffhaltigen Schicht aus der osmotisch aktiven, mit einer wasserpermeabelen Membran umgebenen Darreichungsform. Der Druckausgleich bzw. die Wirkstoff-Freisetzung findet hierbei durch ein Loch in der Membran statt.

Die oben kurz beschriebenen retardiert freisetzenden Darreichungsformen sind jedoch nur für Wirkstoffe anwendbar die effektiv in allen Bereichen des Gastrointestinaltraktes absorbiert werden. Für Wirkstoffe, die auf Grund ihrer physikochemischen Eigenschaften oder bedingt durch mikrobiellen Abbau sgn. Absorptionsfenster aufweisen, d.h. nur in bestimmten Bereichen des Gastrointestinaltraktes (GI-trakt) absorbiert werden, sind sie nicht geeignet, weil sie den Magen-Darm Trakt kontinuierlich durchlaufen und ihre Verweilzeit im absorbierenden Teil des GI-traktes somit zu kurz ist, um eine Langzeitwirkung zu gewährleisten. Beispiele für Substanzen, deren Bioverfügbarkeit stark von der lokalen Physiologie im GI-Traktes abhängt und die vorzugsweise in den höheren Darmabschnitten absorbiert werden, sind Ciprofloxacin und Nimodipin. Ciprofloxacin ist in der sauren Umgebung des Magens gut löslich. Im Darm, wo neutrale bis leicht alkalische pH-Bedingungen vorherschen, kommt es jedoch zur Ausfällung des Wirkstoffes, was die Absorption in den tieferen Darmabschnitten negativ beeinflußt. Nimodipin wird durch die im Colon vorherrschende Bakterien-Flora abgebaut, und kann deshalb nur im oberen Darmbereich effektiv absorbiert werden. Weitere Wirkstoffe, die Absorptionsfenster im oberen GI-Trakt aufweisen sind Captopril und Ranitidin. Wieder andere Wirkstoffe, wie beispielsweise bestimmte Antacida und Pepstatin sind lokal wirksam und können ihre Wirkung nur entfalten, wenn sie am Wirkort, dem Magen, freigesetzt werden.

Im Hinblick auf die obigen Ausführungen wird klar, daß eine große Zahl von Wirkstoffen für herkömmliche Retardformulierungen nicht geeignet sind und daß Bedarf an Systemen besteht, die über längere Zeit im Magen verweilen und dort den Wirkstoff kontrolliert abgeben.

In der Patentliteratur sind unterschiedliche Ansätze zur Verlängerung der Verweilzeit, die auf quellbare oder schwimmfähige Darreichungsformen beruhen, beschrieben. So beschreiben beispielsweise US 3574820 und US 4434153 Tabletten, die im Magen quellen und dadurch so groß werden, daß sie bedingt durch ihre Sperrigkeit den Pförtner nicht mehr passieren können.

Neben der Sperrigkeit wird auch die Herabsetzung der Dichte als Maßnahme zur Verlängerung der Magenverweilzeit genutzt. Darreichungsformen deren Dichte geringer ist als die des Mageninhaltes schwimmen auf und werden somit vom Magenausgang, der sich im unteren Bereich des Magens befindet, ferngehalten. JP 62283919 beschreibt beispielsweise eine Tablette aus einem wirkstoffhaltigen Teil und einem Teil der eine gasbildende Mischung enthält. In Kontakt mit wäßrigen Medien bildet sich Kohlensäure, wodurch die mittlere Dichte der Darreichungsform herabsetzt wird und die Tablette auf dem Mageninhalt aufschwimmt. Die Herabsetzung der Dichte durch Gasbildung wurde auch, wie in EP-A 0235718 beschrieben, bei Granulaten aus einem Wirkstoff und einer gasbildende Mischung, beschichtet mit einer permeabelen dehnbaren Lackschicht, angewandt. Die obengenannten Effekte der Sperrigkeit und Dichtereduktion durch Gasbildung werden auch, wie in der japanischen Patentanmeldung 284093/91 dargestellt, kombiniert eingesetzt.

Andere in der Patentliteratur beschriebenen Darreichungsformen sind nicht auf Gasbildung zur Reduzierung der Dichte angewiesen und besitzen bereits durch ihren Aufbau bei der Applikation eine Dichte, die die Schwimmfähigkeit auf dem Mageninhalt gewährleistet. So beschreibt US 3976764 Systeme in den unterschiedlichsten Ausführungsformen, die so aufgebaut sind, daß ein hohler Kern, bzw. ein Kern geringer Dichte mit einer Wirkstoffhaltigen Schicht beschichtet wurde. In Anlehnung daran beschreibt EP 0326816 Darreichungsformen in denen die Herabsetzung der Dichte durch die Verwendung von Strukturelementen mit Hohlräumen wie Schäume oder Hohlkörper erreicht wird. In US 4167558 werden Darreichungsformen beschrieben, die im wesentlichen aus einer Mischung aus Wirkstoff und gelbildenden Polymeren in einer Kapsel bestehen. Im Magen bildet sich nach Auflösen der Kapsel durch Anquellen der Pulvermischung ein Gelkörper, der noch trockene Pulvermischung enthält. Der Wirkstoff wird im Laufe der Zeit durch Erosion des Gelmantels freigesetzt. Durch weiteres Anquellen des trockenen Kernes wird dabei immer wieder neues Gel gebildet. Die Darreichungsform bleibt solange schwimmfähig bis die ganze Pulvermasse durchfeuchtet ist.

Trotz der Vielfältigkeit der Ansätze zur Verlängerung der Magenverweilzeit stellt sich ihre Umsetzung in praktische Arzneiformen aus den verschiedenen Gründen schwierig dar. Reine Schwimmarzneiformen wie die oben dargelegten Pellets (EP-A-0235718) oder Systeme mit inherenter geringer Dichte (US 3976764, EP-A-0326816 und US 4167558), können nur über längere Zeit im Magen verweilen, wenn der Magen Nahrung enthält. Im nüchternen Zustand verlassen solche Darreichungsformen, bedingt durch ihre relativ geringe Größe den Magen innerhalb kurzer Zeit. Zudem sind die genannten Darreichunsgformen mit geringer inherenter Dichte, bedingt durch ihren bezogen auf das Volumen der Gesamtdarreichungsform relativ geringen Wirkstoffgehalt nur für niedig dosierte Wirkstoffe geeignet. Die in EP 0235718 B1 beschriebenen Granulate sind wiederum nur für wasserlösliche Wirkstoffe anwendbar, was ihren Einsatzbereich stark einschränkt.

Die quellfähigen Tabletten, beschrieben in US 3574820, US 4434153 sowie in der JP 284093/91 kranken an den Eigenschaften der verwendeten Quellmatrix. Eine gute Quellmatrix kann das Vielfache ihres ursprünglichen Gewichtes an Feuchtigkeit aufnehmen. Gleichzeitig muß sie jedoch auch im gequollenen Zustand eine gewisse Formstabilität aufweisen, um der mechanischen Belastung, der sie im Magen unterliegt, stand zu halten. Normale lineare Gelbildner wie modifizierte Cellulosen, Polyoxyethylen und Polyvinylpyrrolidon (PVP), oder auch quervernetzte Polymerisate, beispielsweise auf der Basis von PVP bzw. Polyacrylate zeichnen sich zwar durch gute Flüssigkeitsaufnahme aus, ihre Formstabilität nimmt jedoch wegen des schlechten Verbundes zwischen den hydratierten linearen Polymerketten bzw. vernetzen Polymerpartikeln mit zunehmenden Quellungsgrad stark ab, was zur Erosion bzw. zum Zerfließen der Gelschicht führt. In JP 284093/91 wurde deshalb die Darreichungsform mit einem wasserdurchlässigen, expansionsfähigen Lack überzogen. Dieses Verfahren ist jedoch aufwendig und beschränkt seine Anwendung auf wasserlösliche Wirkstoffe.

Wesentlich bessere mechanische Eigenschaften haben Gele, die als Comprimat, wie in US 3574820 beschrieben, aus völlig vernetzten Polymeren bestehen. Da es sich jedoch bei diesen vernetzten Polymerisaten um neue Hilfsstoffe ohne Zulassung handelt ist ihre Anwendung nicht absehbar.

Es sind auch Beispiele für Gele bekannt, bei denen die zur Gelbildung führende Vernetzung nicht auf kovalente Bindungen, sondern auf physikalisch chemischen Wechselwirkungen zwischen verschiedenen Polymeren beruht. So beschreibt US 3634584 Gele aus Mischungen aus Carboxyvinylpolymeren und Polyethylenglycol und US 3458622 die Verwendung von Mischungen aus PVP und Carboxyvinylpolymeren bei der Bereitstellung von Darreichungsformen mit kontrollierter Wirkstoff-Freisetzung. Die beschriebenen Gele zeichnen sich jedoch nicht durch eine ausgeprägte mechanische Stabilität aus, was schon daraus hervorgeht, daß sie als Erosionsmatrix für die kontrollierte Freigabe von Wirkstoffen genutzt werden. Zudem weisen diese Gele keine ausgeprägten Quelleigenschaften auf. JP 334292 beschreibt Gele aus amidgruppenhaltigen Polymeren und N-substituierten Lactamen als Enzymträgersysteme. Auch diese Gele sind mechanisch nicht beanspruchbar, da sie sich in größeren Volumina wäßriger Medien auflösen.

Der Stand der Technik belegt im allgemeinen die Schwierigkeit der Bereitstellung von Darreichungsformen mit verlängerter Magenverweilzeit und im besonderen die Schwierigkeit der Verwendung von quellfähigen Darreichungsformen auf der Basis pharmazeutisch akzeptabeler Polymere, die im gequollenen Zustand eine hohe Formstabilität aufweisen und bedingt durch ihre Sperrigkeit über längere Zeit im Magen verweilen.

Gegenstand der vorliegenden Erfindung sind Darreichungsformen mit längerer Magenverweilzeit, bei deren Bereitstellung Mischungen aus lactamgruppenhaltigen Polymeren und carboxylgruppenhaltigen Polymeren verwendet werden, die sich sowohl durch ausgeprägte Quelleigenschaften als auch durch hohe Formstabilität im gequollenem Zustand auszeichnen.

Es wurde gefunden, daß Darreichungsformen, die neben Wirkstoff (I), und in der Pharmazie gängigen Hilfsstoffen (II), als Gelbildner Mischungen aus lactamgruppenhaltigen Polymeren (III) und carboxylgruppenhaltigen Polymeren (IV) enthalten, in Medien mit saurem pH-Wert, wie er typischerweise im Magen vorliegt, das vielfache ihres ursprünglichen Gewichtes an Wasser aufnehmen, und im Gegensatz zu Darreichungsformen die vergleichbare Mengen eines herkömmlichen Gelbildners enthalten, zu formstabilen Gelen anquellen. Vorteilhaft ist dabei wenn im Gelbildner die Polymere(III) und (IV) in intensiv gemischter Form vorliegen. Ggf. sind in den Darreichungsformen zudem gasbildende Agentien (V) enthalten.

Die im Gelbildner enthaltenen lactamgruppenhaltigen Polymere (III) sind Verbindungen auf der Basis von Vinyllactamen wie Vinylcaprolactam und Vinylpyrrolidon, die bezogen auf die bei der Herstellung eingesetzten Vinylmonomere in Mengen zwischen 20 und 100 Gewichts-%, vorzugsweise jedoch zwischen 80 und 100 Gewichts-% enthalten sind.

Besonders bevorzugte lactamgruppenhaltige Polymere im Sinne der Erfindung sind lineare Polyvinylpyrrolidone mit hohem Molekulargewicht, wie sie beispielsweise unter dem Hadelsnahmen "LUVISKOL K90® " bzw. "KOLLIDON K90® " von der BASF vertrieben werden.

Die im Gelbildner enthaltenen carboxylgruppenhaltigen Polymere (IV) sind solchen Polymere mit titrierfähigen Carboxylgruppen, die eine Säurezahl zwischen 50 und 1200 mg KOH / g, vorzugsweise jedoch zwischen 300 und 1000 mg KOH / Festsubstanz Polymer aufweisen. Die Säurezahl gibt an, wieviel mg KOH zur Neutralisation der in 1 g Polymertrockensubstanz enthaltenen sauren Gruppen notwendig sind. Beispiele solcher Polymere sind Carboxymethylcellulose, Alginate oder synthetische Polymere auf der Basis von Vinylmonomeren wie Acrylsäure, Methacrylsäure, Maleinsäure oder Fumarsäure.

Besonders bevorzugte carboxylgruppenhaltige Polymere im Sinne der Erfindung sind carboxylgruppenhaltige Acrylharze, wie sie von der Röhm Pharma GmbH unter dem Handelsnamen "EUDRAGIT® " vertrieben werden. Besonders bevorzugte Eudragit-Sorten sind "EUDRAGIT L® " und "EUDRAGIT S® ".

In einer bevorzugten Ausführungsform ist das carboxylgruppenhaltige Polymer (IV) ein Copolymerisat auf der Basis von Methacrylsäure und Methylmethacrylat, mit einem Verhältnis der freien Carboxylgruppen zu den Estergruppen 1:2, vorzugsweise jedoch 1:1.

In den erfindungsgemäßen Darreichungsformen sind die lactamgruppenhaltigen Polymere (III) und die carboxylgruppenhaltigen Polymere (IV) in einem Mischungsverhältnis zwischen 40:60 und 98:2, vorzugsweise jedoch zwischen 80:20 und 95:5 vorhanden. Die Quelleigenschaften der Darreichungsformen, sowie ihre mechanische Stabilität im gequollenen Zustand wird dabei maßgeblich durch das Mischungsverhältnis der beiden Polymere beeinflusst.

Die intensive Vermischung der Polymere (III) und (IV), die letztendlich wesentlich die guten Quelleigenschaften und die mechanische Stabilität der daraus hergestellten Darreichungsformen bestimmt, kann beispielsweise durch Trocknung von Mischlösungen, die die erfindungsgemäßen Polymere enthalten, gewährleistet werden. Hierbei wird das carboxylgruppenhaltige Polymer in seiner neutralisierten Form vorgelegt, um Gelbildung in der Mischlösung zu vermeiden. Bei einer anderen Methode zur Herstellung von Polymermischungen wird eine Pulvermischung der Polymere, ggf. versehen mit Wirkstoff und / oder anderen in der pharmazeutischen Technologie gängigen Hilfsstoffen wie Schmiermittel, Bindemittel, Füllstoffe usw. mittels geeigneter Verfahren, z. B. Tablettierung, zu Formkörpern gewünschter Geometrie verarbeitet, und dann bei einer Temperatur, dir mindestens überhalb der Glastemperatur einer der Polymere liegen sollte, über längere Zeit gelagert. Diese Lagerung bewirkt durch Interpenetration der unterschiedlichen Polymere einen intensiven molekularen Verbund.

Hilfsstoffe (II) im Sinne der Erfindung sind solche, die zur Gewährleistung der Herstellbarkeit der Darreichungsformen bzw. ihrer Eigenschaften wie Härte, Abrieb usw. notwendig sind. Hierunter fallen beispielsweise die in der pharmazeutischen Praxis gängigen, dem Fachkundigen bekannten Fließverbesserer, Füllstoffe, Schmier- und Bindemittel.

Als Wirkstoffe oder Wirkstoffkombinationen (I) gemäß vorliegender Erfindung kommen alle in Frage, welche zur oralen Applikation und zur Retard-Therapie geeignet sind. Voraussetzung ist jedoch, dass es sich hierbei um nicht säureempfindliche Wirkstoffe handelt. Wirkstoffe schließen ein: Ciprofloxacin, Nimodipin, Captopril, Ranitidin, Ciclosporin, Baclofen, Allopurinol, Furosemid, Cefoxitin, 5-Aminosalicylat oder Moexipril. Besonders geeignet sind die erfindungsgemäßen Darreichungsformen für Wirkstoffe, die ihr Absorptionsfenster im Magen bzw. im oberen Teil des Gastrointestinaltraktes aufweisen wie Ciprofloxacin, Nimodipin, Captopril und Ranitidin, oder aber dort lokal ihre Wirkung entfalten wie bestimmte Antacida. Beispiele solcher Antacida sind Magnesiumhydroxid und Magnesiumtrisilikat.

Als gasbildende Agentien (V) die ggf. zur Erhöhung des Auftriebes eingesetzt werden, kommen alle Substanzen in Frage die in Kontakt mit Wasser bzw. Magenflüssigkeit in der Lage sind, nicht-toxische Gase zu bilden. Beispiele sind Hydrogencarbonate wie beispielsweise Natriumhydrogencarbonat die einzeln, oder in Kombination mit Säuren eingesetzt werden. Beispiele solcher Säuren sind Zitronensäure oder auch Polyacrylate wie sie durch die Firma B.F. Goodrich Chemical GmbH unter dem Namen "CARBOPOL® " vertrieben werden. Das sich bildende Gas wird als Blasen in der hydratisierten Gelschicht eingelagert und trägt so zum Auftrieb der Tablette bei.

Die Verhältnisse der in den erfindungsgemäßen Darreichungsformen enthaltenen Mengen Gelbildner (III) und (IV) zu Wirkstoff (I) bzw. Hilfsstoffe (II) unterliegen keinen Beschränkungen. Sie hängen von der Wirkstoff-Dosierung, der Art des Wirkstoffes und dem Aufbau der Darreichungsform ab. Wesentlich ist jedoch, daß die Darreichungsform soviel Gelbildner enthält, daß sie nach Applikation bis zu einer Größe quellen kann, die einen Durchgang durch den Pylorus über längere Zeit verhindert.

Desgleichen kann auch der in den erfindungsgemäßen Darreichungsformen enthaltene Wirkstoffanteil je nach Art des Wirkstoffes, Grad der gewünschten Retardierung der Wirkstoff-Freisetzung und Ausführungsart der Darreichungsform sehr unterschiedlich sein.

Die erfindungsgemäßen Darreichungsformen sind in den unterschiedlichsten Ausführungsformen wie Tabletten, Kapseln und Granulaten bzw. Pellets herstellbar. Besonders vorteilhaft ist jedoch die Ausführungsform der Tablette. So kann beispielsweise der Wirkstoff, zusammen mit den erfindungsgemäßen Polymeren und ggf. anderen in der pharmazeutischen Technologie gängigen Hilfsstoffen, zu einer homogenen Tablette verpreßt werden. Besonders bevorzugt sind jedoch Systeme in einer Form, in der der Wirkstoff und die Polymere (III) und (IV) räumlich von einander getrennt vorliegen, wie es beispielsweise bei Kern-Mantel Tabletten mit azentrischem, an einer Fläche freiliegenden Kern sowie bei Doppelschicht-Tabletten der Fall ist. Ggf. kann in den Darreichungsformen auch eine gasbildende Mischung eingearbeitet werden, um die quellungsbedingte Verlängerung der Verweilzeit im Magen durch zusätzlichen Auftrieb zu unterstützen.

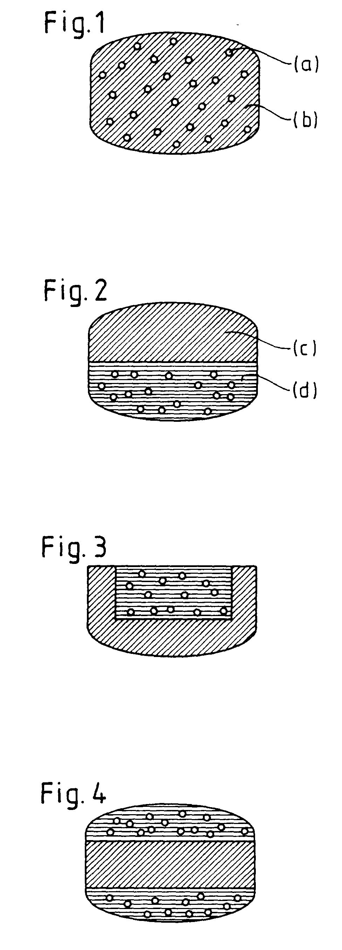

Die Abbildungen 1 bis 4 zeigen, schematisch als Querschnittzeichnung dargestellt, Beispiele unterschiedlicher Ausführungsformen der erfindungsgemäßen Darreichungsformen. Abbildung 1 stellt eine Darreichungsform dar, in der der Wirkstoff (a) molekular gelöst, oder aber in disperser Form in einen Gelbildner (b) auf der Basis von Mischungen aus lactamgruppenhaltigen (III) und carboxylgruppenhaltigen (IV) Polymeren eingelagert ist. Der Gelbildner in den dargestellten und unten genannten Ausführungsarten kann zusätzlich zum Wirkstoff gasbildende Agentien (V) enthalten. In der in Abbildung 2 wiedergegebenen Darreichungsform liegen die gelbildende quellfähige Schicht (c), und die den Wirkstoff enthaltende Schicht (d) von einander getrennt, in der Form einer Doppelschicht-Tablette vor. Wie in Abbildung 3 und 4 beispielhaft dargestellt, ist eine räumliche Trennung der beiden Schichten auch mit anderen Ausführungsformen zu erreichen. Je nach Löslichkeit des Wirkstoffes ist die eine oder andere Ausführungsform vorzuziehen. So eignet sich eine Darreichsform gemäß Abbildung 1 besonders für magensaftlösliche Wirkstoffe, für unlösliche Wirkstoffe sind die anderen Ausführungsformen besser geeignet. Alle Ausführungsformen ist jedoch gemeinsam, daß sie im Magen stark quellen, und bedingt dadurch über längere Zeit im Magen verweilen.

Die folgenden Beispiele illustrieren die Erfindung:

**Beispiel 1**

Herstellung des Gelbildners und der Quellkörper: Als Grundlage zur Herstellung des Gelbildners dienten Lösungen des Polyvinylpyrrolidons Luviskol K 90® (lactamgruppenhaltiges Polymer) und Eudragit L® , ein Copolymerisat auf der Basis von Methacrylsäure und Methylmethacrylat (carboxylgruppenhaltiges Polymer). 950 g einer 17.5 Gew.-%igen Lösung von Luviskol K 90® in Wasser wurden mit 50 g einer 17.5 %-igen, mit Ammoniak auf pH 7 eingestellten wäßrigen Lösung von Eudragit L® vermischt. Die Mischlösung wurde gefriergetrocknet, das erhaltene Lyophilisat gemahlen und auf 500 Mikrometer gesiebt. Aus diesem Material wurden mittels einer gängigen Exzenterpresse bei einer Preßkraft von 10 kN Tabletten mit einem Gewicht von 400 mg und einem Durchmesser von 12 mm hergestellt.

Bestimmung des Quellverhaltens: Die Tabletten wurden in 0.1 N HCl bei einer Temperatur von 37°C inkubiert. Zu bestimmten Zeitpunkten wurden die Tabletten dem Inkubationsmedium entnommen, die anhaftende Flüssigkeit entfernt und das Gewicht der gequollenen Tabletten bestimmt. Der Quellgrad (Qt) zum Zeitpunkt t wurde definiert als der Quotient des Gewichtes der Tablette zum Zeitpunkt t (Wt) und dem Trockengewicht der Tablette (Wo):

$$Qt = Wt /Wo$$

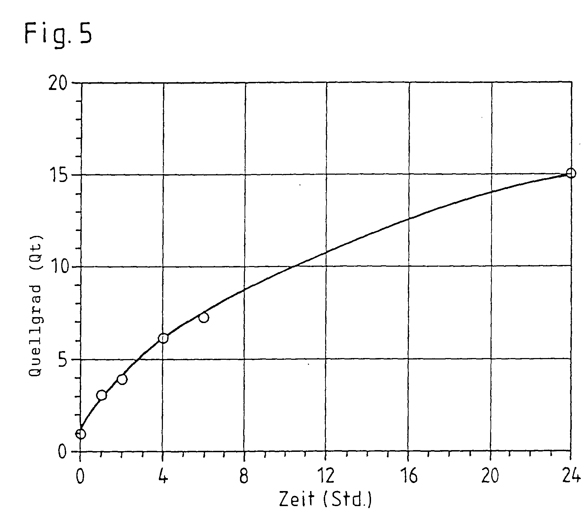

Der Verlauf des Quellgrades der beschriebenen Tablette als Funktion der Zeit ist in Abbildung 5 grafisch dargestellt.

Bestimmung der mechanischen Eigenschaften: Stabile Gele zeichnen sich dadurch aus, daß sie während des Quellvorganges zwar an Volumen zunehmen, dabei jedoch ihre ursprüngliche Form beibehalten (Formstabilität). Zudem reagieren sie auf mechanische Verformung reversibel, und nehmen nach Wegnahme der Verformungskraft ihre ursprüngliche Form wieder ein (hohe Elastizität). Gele der beschriebenen Zusammensetzung zeichnen sich durch hohe Formstabilität und gutes elastisches Verhalten aus.

**Vergleichsbeispiel 2**

Es wurden Tabletten hergestellt mit der gleichen Zusammensetzung wie in Beispiel 1, wobei die gelbildenden Komponenten jedoch nicht als molekulare Mischung, hergestellt über Lösungen, sondern als reine Pulvermischung verarbeitet wurden. Die Tabletten wiesen in 0.1 N HCl schlechte mechanische Eigenschaften auf. Sie waren stark erosionsempfindlich und lösten sich im Laufe der Zeit vollständig auf. Eine Bestimmung der Quellgrades (Qt) als Funktion der Zeit, gemäß Beispiel 1 war wegen der nicht ausreichenden mechanischen Festigkeit nicht möglich.

**Beispiel 3**

Gemäß Beispiel 1 wurden Tabletten mit unterschiedlichen Verhältnissen Luviskol K 90® und Eudragit L® hergestellt und ihr Quellgrad (Qt) gemäß Beispiel 1 nach 24 Std. bestimmt. Die Ergebnisse sind in der folgenden Tabelle zusammengefaßt.

| Gew.- Verhältnis Luviskol K 90®: Eudragit L® | Quellgrad Qt nach 24 Std |
|---|---|
| 90:10 | 10.4 |

(fortgesetzt)

| Gew.- Verhältnis Luviskol K 90®: Eudragit L® | Quellgrad Qt nach 24 Std |
|---|---|
| 92.5:7.5 | 12.7 |
| 95:5 | 15.3 |
| 96:4 | 17.2 |
| 97:3 | 18.2 |
| 98:2 | 20.4 |
| 100:0 | * |

*) keine Gelbildung, Tablette löst sich vollständig auf.

Aus diesen Daten ist ersichtlich, daß sich das Quellverhalten der Gele durch das Verhältnis Luviskol K 90® : Eudragit L® steuern läßt. Die Gele weisen mit zunehmenden Eudragit L® -Gehalt eine zunehmende Formstabilität und Elastizität auf. Gele bilden sich nur aus, wenn beide polymeren Komponenten vorliegen. Reines PVP ist kein beständiger Gelbildner.

**Vergleichsbeispiel 4**

Aus den in der nachfolgenden Tabelle aufgeführten bekannten Gelbildnern wurden Tabletten mit gleichem Gewicht und Abmessungen wie in Beispiel 1 gepreßt und ihr Quellgrad (Qt) nach 24 Std. gemäß Beispiel 1 bestimmt.

| Polymer | Typ | Hersteller | Quellgrad (Qt) nach 24 Std. |
|---|---|---|---|
| Polyoxyethylen | Polyox Coagulant® | Union Carbide | 11.7 |
| Carboxymethylcellulose | Tylose C 6000® | Hoechst | * |
| Hydroxyethylcellulose | Tylose H 10000® | Hoechst | 10.3 |
| Polyacrylat | Carbopol 974 P® | B.F. Goodrich | 9.4 |

*) Tablette zerfällt innerhalb einer Stunde

Die genannten Gelbildner haben alle schlechte mechanische Eigenschaften Sie sind stark erosionsempfindlich und verformen sich bereits bei geringer mechanischer Belastung irreversibel. Dahingegen sind die im Beispiel 3 beschriebenen Gele mit vergleichbarem, oder besserem Quellverhalten ( z.B. Gele auf der Basis von 92.5 Teilen PVP K 90 und 7.5 Teilen Eudragit L), erosionsunempfindlich und weisen durch ihre hohe Elastizität bei mechanischer Belastung keine bleibende Verformung auf.

**Beispiel 5**

Herstellung einer Monoschicht-Tablette gemäß Abbildung 1: Auf einer Exzenterpresse wurden bei einer Preßkraft von 10 kN 12 mm Tabletten folgender Zusammensetzung hergestellt:

| Einsatzstoff | Menge (mg /Tablette) |
|---|---|
| Ciprofloxacin-HCl | 250 |
| Gelmischung hergestellt gemäß Beispiel 1 (Luviskol K 90®: Eudragit L®=95:5) | 229.8 |
| Natriumbicarbonat Pulver | 20.2 |

Die Wirkstoff-Abgabe der Tabletten wurde in einer gängigen Freisetungsapparatur bestimmt. Innerhalb von 24 Std. setzten die Tabletten in 900 ml 0.1 N HCl bei 37°C unter stetem Rühren (75 Umdrehungen pro Minute) den Wirkstoff vollständig frei. Sie nahmen dabei soviel Flüssigkeit auf, daß ihr Durchmesser bei guter mechanischer Stabilität gemäß der bei Beispiel 1 genannten Kriterien 3 cm betrug.

**Beispiel 6**

Herstellung einer Doppelschicht-Tablette gemäß Abbildung 2: Nach der nachfolgend dargestellten Rezeptur wurden durch Verpressung auf einer Exzenterpresse bei einem Preßdruck von 1.5 Tonnen Doppelschicht-Tabletten mit einem Durchmesser von 12 mm bestehend aus einer Quellschicht und einer Wirkstoffhaltigen Schicht hergestellt:

Die Wirkstoff-Abgabe der Tabletten wurde in einer gängigen Freisetungsapparatur der Firma ERWEKA bestimmt. Innerhalb von 7 Std. setzen die Tabletten in 900 ml 0.1 N HCl bei 37°C unter stetem Rühren (75 Umdrehungen pro Minute) 90 % des Wirkstoffes linear frei. Die wirkstoffhaltige Schicht löste sich dabei nahezu vollständig auf. Die Quellschicht dahingegen nahm im Laufe der Zeit stark im Volumen zu. Der Durchmesser betrug nach 6 Std. 2 cm, nach 24 Std. 3 cm. Die Gelschicht zeichnete sich auch nach 24 Std. durch gute mechanische Eigenschaften aus. Sie war bei mechanischer Belastung reversibel verformbar und wies keine sichtbare Erosion auf. Bedingt durch Gasbildung durch das in der Gelschicht enthaltene Natriumbicarbonat trieb die Tablette während der Freisetzung an der Oberfläche des Freisetzungsmediums.

| Einsatzstoff | Menge (mg /Tablette) |
|---|---|
| Quellschicht | |
| Gelmischung hergestellt gemäß Beispiel 1 (Luviskol K 90®: Eudragit L® = 95:5) | 340 |
| Carbopol 974 P | 34 |
| Magnesiumstearat | 10 |
| Eisenoxid rot | 2.5 |
| Natriumbicarbonat | 34 |
| Wirkstoffhaltige Schicht | |
| Ciprofloxacin HCl | 250 |
| Magnesiumstearat | 2.9 |
| Klucel JF® (Hydroxypropylcellulose) | 80 |

**Patentansprüche**

1. Darreichungsformen mit verlängerter Magenverweilzeit, enthaltend

   (I) Wirkstoff oder eine Kombination mehrerer Wirkstoffe,

   (II) andere in der Pharmazie gängige Hilfsstoffe,
   einen Gelbildner aus einer homogenen Mischung aus lactamgruppenhaltigen Polymeren (III) und carboxylgruppenhaltigen Polymeren (IV) und ggf.

   (V) gasbildende Zusatzstoffe,

   **dadurch gekennzeichnet, daß** sie sich in sauren wäßrigen Medien durch ausgeprägte Quelleigenschaften und hohe mechanische Stabilität im gequollenen Zustand kennzeichnen.

2. Darreichungsformen gemäß Anspruch 1, **dadurch gekennzeichnet, daß** der Gelbildner die lactamgruppenhaltigen und carboxylgruppenhaltigen Polymere (III) und (IV) in molekular gemischter Form vorliegend enthält.

3. Darreichungsformen gemäß Anspruch 1 und 2, **dadurch gekennzeichnet, daß** das Polymer (III) die lactamgruppenhaltigen Monomere, bezogen auf die bei der Herstellung des Polymeren eingesetzten Vinylmonomere in Mengen zwischen 20 und 100 Gewichts-%, vorzugsweise jedoch zwischen 80 und 100 Gewichts-% enthält.

4. Darreichungsformen gemäß Anspruch 1 und 2, **dadurch gekennzeichnet, daß** das lactamgruppenhaltige Polymer (III) ein Polyvinylpyrrolidon mit einem Molekulargewicht > 200.000 im Gewichtsmittel darstellt.

5. Darreichungsformen gemäß Anspruch 1 und 2, **dadurch gekennzeichnet, daß** das carboxylgruppenhaltige Polymer (IV) eine Säurezahl zwischen 100 und 1200 mg KOH / g Festsubstanz Polymer aufweist.

6. Darreichungsformen gemäß Anspruch 1 und 2, **dadurch gekennzeichnet, daß** das carboxylgruppenhaltige Polymer (IV) ein Copolymerisat auf der Basis von Methacrylsäure und Methylmethacrylat, mit einem Verhältnis der freien Carboxylgruppen zu den Estergruppen 1:2, vorzugsweise jedoch 1:1 darstellt.

7. Darreichungsformen gemäß Anspruch 1 bis 6, **dadurch gekennzeichnet, daß** die lactamgruppenhaltigen Polymere (III) und die carboxylgruppenhaltigen Polymere (IV) in einem Mischungsverhältnis zwischen 40:60 und 98:2, vorzugsweise jedoch zwischen 80:20 und 95:5 vorhanden sind.

8. Darreichungsformen gemäß Anspruch 1 bis 6, **dadurch gekennzeichnet, daß** die darin enthaltenen Wirkstoffe oder Wirkstoffkombinationen (I) ein Absorptionsfenster im Magen bzw. im oberen Teil des Gastrointestinaltraktes aufweisen, oder aber lokal im Magen bzw. oberen Teil des humanen Gastrointestinaltraktes ihre Wirkung entfalten.

9. Darreichungsformen gemäß Anspruch 1 bis 7, **dadurch gekennzeichnet, daß** sie als Wirkstoff Ciprofloxacin, Nimodipin, Captopril, Ranitidin, Ciclosporin, Baclofen, Allopurinol, Furosemid, Cefoxitin, 5-Aminosalicylat oder Moexipril enthalten.

10. Darreichungsformen gemäß Anspruch 1 bis 7, **dadurch gekennzeichnet, daß** der darin enthaltene Wirkstoff ein Antacidum wie Magnesiumhydroxid, Magnesiumtrisilikat oder dergleichen ist.

## Claims

1. Administration forms having a prolonged gastric residence time, containing

   (I) active compound or a combination of two or more active compounds,

   (II) other auxiliaries customary in pharmacy,
   a gel-forming agent comprising a homogeneous mixture of polymers (III) containing lactam groups and polymers (IV) containing carboxyl groups and optionally

   (V) gas-forming additives,

   **characterized in that** they are distinguished in acidic aqueous media by marked swelling properties and high mechanical stability in the swollen state.

2. Administration forms according to Claim 1, **characterized in that** the gel-forming agent contains the polymers (III) and (IV) containing lactam groups and carboxyl groups in molecularly mixed form.

3. Administration forms according to Claims 1 and 2, **characterized in that** the polymer (III) contains the monomers containing lactam groups, based on the vinyl monomers employed in the preparation of the polymer, in amounts between 20 and 100 % by weight, but preferably between 80 and 100 % by weight.

4. Administration forms according to Claims 1 and 2, **characterized in that** the polymer (III) containing lactam groups is a polyvinylpyrrolidone having a weight-average molecular weight > 200,000.

5. Administration forms according to Claims 1 and 2, **characterized in that** the polymer (IV) containing carboxyl groups has an acid number between 100 and 1,200 mg of KOH/g of polymer solid substance.

6. Administration forms according to Claims 1 and 2, **characterized in that** the polymer (IV) containing carboxyl groups is a copolymer based on methacrylic acid and methyl methacrylate, having a ratio of the free carboxyl groups to the ester groups of 1:2, but preferably 1:1.

7. Administration forms according to Claims 1 to 6, **characterized in that** the polymers (III) containing lactam groups and the polymers (IV) containing carboxyl groups are present in a mixing ratio of between 40:60 and 98:2, but preferably between 80:20 and 95:5.

8. Administration forms according to Claims 1 to 6, **characterized in that** the active compounds or active compound combinations (I) contained therein have an absorption window in the stomach or in the upper part of the gastrointestinal tract, or else display their action locally in the stomach or upper part of the human gastrointestinal tract.

9. Administration forms according to Claims 1 to 7, **characterized in that** as active compound they contain ciprofloxacin, nimodipine, captopril, ranitidine, cyclosporin, baclofen, allopurinol, furosemide, cefoxitine, 5-aminosalicylate or moexipril.

**10.** Administration forms according to Claims 1 to 7, **characterized in that** the active compound contained therein is an antacid such as magnesium hydroxide, magnesium trisilicate or the like.


**Revendications**

**1.** Formes d'administration avec temps de séjour prolongé dans l'estomac, contenant :

(I) un agent actif ou une combinaison de plusieurs agents actifs ;
(II) d'autres additifs usuels en pharmacie,
un agent formant gel constitué d'un mélange homogène de polymères contenant des radicaux lactames
(III) et de polymères contenant des radicaux carboxyle (IV), et facultativement
(V) des additifs formant un gaz,

**caractérisées en ce qu'**elles se distinguent par des propriétés marquées de gonflement en milieu aqueux acide et par une stabilité mécanique élevée à l'état gonflé.

**2.** Formes d'administration suivant la revendication 1, **caractérisées en ce que** l'agent formant gel contient les polymères contenant des radicaux lactames (III) et contenant des radicaux carboxyle (IV) sous forme de mélange moléculaire.

**3.** Formes d'administration suivant les revendications 1 et 2, **caractérisées en ce que** le polymère (III) contient les monomères contenant des radicaux lactame en des quantités allant de 20 à 100% en poids, mais de préférence de 80 à 100% en poids, sur base des monomères vinyliques mis en oeuvre dans la préparation des polymères.

**4.** Formes d'administration suivant les revendications 1 et 2, **caractérisées en ce que** le polymère contenant des radicaux lactame (III) est une polyvinylpyrrolidone avec un poids moléculaire moyen en poids > 200 000.

**5.** Formes d'administration suivant les revendications 1 et 2, **caractérisées en ce que** le polymère contenant des radicaux carboxyle (IV) présente un indice d'acide allant de 100 à 1200 mg KOH/g de matière solide polymère.

**6.** Formes d'administration suivant les revendications 1 et 2, **caractérisées en ce que** le polymère contenant des radicaux carboxyle (IV) est un copolymère à base d'acide méthacrylique et de méthacrylate de méthyle, avec un rapport des radicaux carboxyle libre aux radicaux ester de 1:2, mais de préférence de 1:1.

**7.** Formes d'administration suivant les revendications 1 à 6, **caractérisées en ce que** le polymère contenant des radicaux lactame (III) et le polymère contenant des radicaux carboxyle (IV) sont présents en un rapport des ingrédients allant de 40:60 à 98:2, mais de préférence de 80:20 à 95:5.

**8.** Formes d'administration suivant les revendications 1 à 6, **caractérisées en ce que** l'agent actif ou la combinaison d'agents actifs (I) qu'elles contiennent, présente une fenêtre d'absorption dans l'estomac ou la partie supérieure du tractus gastro-intestinal, ou développe son activité de manière locale dans l'estomac ou la partie supérieure du tractus gastro-intestinal humain.

**9.** Formes d'administration suivant les revendications 1 à 7, **caractérisées en ce qu'**elles contiennent comme agent actif, la ciprofloxacine, la nimodipine, le captopril, la ranitidine, la ciclosporine, le baclofène, l'allopurinol, le furosémid, la céfoxitine, le 5-aminosalicylate ou le moexipril.

**10.** Formes d'administration suivant les revendications 1 à 7, **caractérisées en ce que** l'agent actif qu'elles contiennent est un antiacide comme l'hydroxyde de magnésium, le trisilicate de magnésium ou similaire.

Fig.1

(a)

(b)

Fig. 2

(c)

(d)

Fig. 3

Fig. 4

Fig. 5